(19) Europäisches Patentamt
European Patent Office
Office européen des brevets

(11) **EP 2 353 641 B1**

(12) **FASCICULE DE BREVET EUROPEEN**

(45) Date de publication et mention de la délivrance du brevet:
**23.01.2013 Bulletin 2013/04**

(21) Numéro de dépôt: **10192940.4**

(22) Date de dépôt: **29.11.2010**

(51) Int Cl.:
*A61N 1/362* *(2006.01)*    *A61N 1/365* *(2006.01)*
*G05B 6/02* *(2006.01)*    *G05B 11/01* *(2006.01)*
*G05B 13/02* *(2006.01)*    *A61N 1/368* *(2006.01)*

(54) **Dispositif médical implantable actif de resynchronisation cardiaque à optimisation automatique et en temps réel des délais interventriculaire et atrioventriculaire**

Implantierbare aktive medizinische Vorrichtung zur Resynchronisation des Herzens mit automatischer Echtzeit-Optimierung der interventrikulären und atrioventrikulären Verzögerungen

Active implantable medical device for cardiac resynchronisation with automatic, real-time optimisation of the interventricular and atrioventricular delays

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.02.2010 FR 1050876**

(43) Date de publication de la demande:
**10.08.2011 Bulletin 2011/32**

(73) Titulaire: **Sorin CRM SAS**
**92143 Clamart Cedex (FR)**

(72) Inventeur: **Makdissi, Alaa**
**75011, PARIS (FR)**

(74) Mandataire: **Dupuis-Latour, Dominique**
**Bardehle Pagenberg**
**10, boulevard Haussmann**
**75009 Paris (FR)**

(56) Documents cités:
WO-A1-2008/133552    US-A1- 2004 078 058
US-A1- 2005 131 469    US-A1- 2007 066 905
US-A1- 2009 105 777    US-B1- 6 522 923
US-B1- 6 792 310    US-B1- 7 558 627

## Description

L'invention concerne les "dispositifs médicaux implantables actifs" tels que définis par la directive 90/385/CEE du 20 juin 1990 du Conseil des communautés européennes.

Elle concerne plus précisément les implants permettant de surveiller en continu le rythme cardiaque et délivrer si nécessaire au coeur des impulsions électriques permettant d'assurer une stimulation conjointe et permanente des ventricules gauche et droit afin de resynchroniser ces derniers, technique dite "CRT" (*Cardiac Resynchronization Therapy*) ou "BVP" *(Bi-Ventricular Pacing).*

Un stimulateur CRT est par exemple divulgué dans le EP 1 108 446 A1 (ELA Medical), qui décrit un dispositif permettant d'appliquer entre les instants respectifs de stimulation des ventricules gauche et droit un délai dit "délai interventriculaire" (DVV ou VVD) variable, ajusté de manière à resynchroniser la contraction des ventricules avec optimisation fine de l'état hémodynamique du patient.

En effet, une stimulation simultanée des deux ventricules n'est pas toujours optimale, car elle n'aboutit pas forcément à une contraction synchrone des deux ventricules du fait, d'une part, des délais de conduction au sein du myocarde qui ne sont pas les mêmes à droite et à gauche et peuvent dépendre de multiples facteurs et, d'autre part, de l'emplacement de la sonde ventriculaire gauche, selon que celle-ci est une sonde enfilée dans le sinus coronaire ou une sonde épicardique. Il est donc souhaitable d'établir un délai entre les deux stimulations, et d'ajuster ce délai pour resynchroniser la contraction des ventricules et assurer ainsi une optimisation fine de l'hémodynamique. Le DVV pourra être nul, positif (le ventricule gauche étant stimulé après le ventricule droit), ou négatif (le ventricule droit étant stimulé après le ventricule gauche).

Les dispositifs CRT incluent en outre un mode de fonctionnement "double chambre" classique dans lequel le dispositif surveille l'activité ventriculaire après un événement auriculaire spontané (détection d'une onde P de dépolarisation auriculaire) ou stimulé (application d'une impulsion A de stimulation auriculaire). En même temps, le dispositif commence à compter un délai dit "délai atrioventriculaire" ou "délai auriculo-ventriculaire" (DAV ou AVD) tel que si aucune activité spontanée ventriculaire (onde R) n'a été détectée à l'issue de ce délai, alors le dispositif déclenche une stimulation de ce ventricule (application d'une impulsion V).

Des études cliniques ont permis d'observer des résultats souvent spectaculaires pour des patients en insuffisance cardiaque non améliorée par les traitements classiques, dès lors que les paramètres de la thérapie CRT ont été ajustés de façon précise en fonction du patient et de la nature de son trouble de la contraction myocardique, tel que dilatation des cavités cardiaques, faible fraction d'éjection, allongement excessif de la durée du complexe QRS (que ce trouble soit spontané ou induit par une stimulation traditionnelle).

Il est en outre nécessaire de réévaluer ultérieurement ces réglages pour les réajuster éventuellement : en effet, les effets bénéfiques procurés par la thérapie CRT peuvent conduire, à terme, à modifier la configuration primitive et le paramétrage de la stimulation.

La technique de référence pour l'ajustement des paramètres de stimulation CRT est l'évaluation par échocardiographie avec estimation des délais caractéristiques de la systole, en particulier du temps d'ouverture de la valve aortique. Cette procédure doit toutefois être mise en oeuvre en milieu hospitalier et par un personnel qualifié, elle est longue et coûteuse et ne peut pas être appliquée aussi souvent que cela serait utile ou nécessaire sans interférer avec la vie quotidienne du patient, malgré les effets bénéfiques de la thérapie CRT.

Une autre difficulté inhérente à l'évaluation échocardiographique tient au fait qu'elle nécessite de tester successivement plusieurs configurations de stimulation, en déterminant pour chacune un DAV optimal. Le nombre de combinaisons à tester est de ce fait important, et implique une procédure lourde, longue et difficile à gérer, ce qui exclut d'en faire une opération de routine.

Il subsiste de ce fait un besoin réel de disposer d'une technique permettant d'évaluer de façon simple, rapide, automatisée et précise l'incidence des différents paramètres de la stimulation CRT, notamment des délais DAV et DVV, de manière à pouvoir optimiser l'état hémodynamique du patient.

Une méthode automatisée d'optimisation est décrite dans l'article de JM Dupuis et al. : Programming Optimal Atrioventricular Delay in Dual Chamber Pacing Using Peak Endocardial Acceleration : Comparison with a Standard Echocardiographic Procedure, PACE 2003; 26: [Pt. II], 210-213. Cette technique consiste à exécuter un balayage du DAV dans une configuration de stimulation donnée, pour tracer une caractéristique donnant la valeur du pic d'accélération endocardiaque PEA (*Peak Endocardial Acceleration*) en fonction du DAV. La valeur considérée optimale du DAV est celle du point d'inflexion de la caractéristique, point correspondant à une durée de remplissage maximale du ventricule sans troncature de l'onde A (retard minimal entre la fermeture de la valve mitrale et le début du complexe QRS).

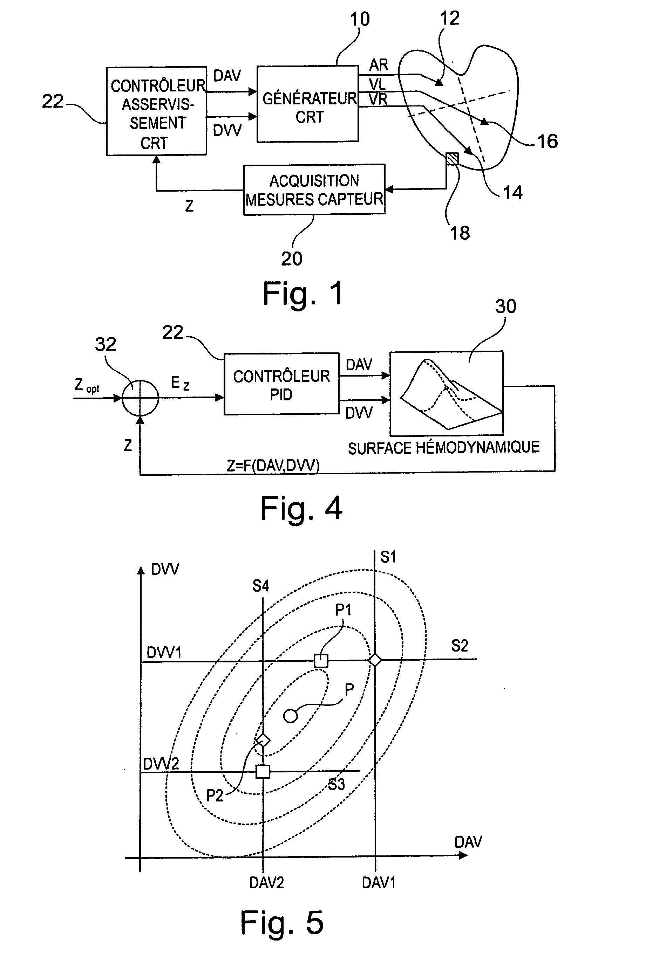

L'algorithme correspondant, s'il donne des résultats satisfaisants, requiert toutefois plusieurs minutes pour son exécution avant de pouvoir sélecttionner le couple {DAV, DVV} optimal, en raison des multiples balayages de DAV pour les diverses valeurs de DVV testées séparément. Un autre inconvénient de cette technique d'optimisation tient au fait que la recherche de chaque délai (DAV ou DVV) se fait indépendamment de l'autre : pour un DVV donné un balayage du DAV donnera un DAV optimal localement, mais (comme on l'expliquera plus en détail par la suite en référence notamment à la Figure 5) la convergence vers un optimum local ne conduit pas nécessairement à l'optimum global. En d'autres termes, le couple {DAV, DVV} optimal ne correspond pas nécessairement à une valeur de DAV optimal à DVV constant, ni à une valeur de DVV optimal à DAV constant.

Les US 6 792 310 B1, US 2007/0066905 A1 et US 2004/0078058 A1 décrivent des techniques d'optimisation comparables, où DAV et DVV sont optimisés de façon indépendante en les faisant varier de façon contrôlée et en examinant l'incidence de cette variation sur la fonction hémodynamique.

Une technique particulière d'optimisation, beaucoup plus rapide et susceptible d'être mise en oeuvre en temps réel, est décrite notamment dans les WO 200/090397 A2 et WO 2006/126185 A2. L'algorithme décrit dans ces documents utilise un réseau neuronal de type *spike* pour identifier le maximum d'une fonction hémodynamique (*stroke volume*). Un réseau *spike* nécessite toutefois un processeur dédié, impliquant donc la conception d'un implant spécifique plus complexe et entraînant une consommation électrique supérieure. Une version logicielle de cet algorithme est certes possible, mais exige dans ce cas des ressources informatiques qui ne sont pas à la portée des microcontrôleurs à ultra-faible consommation tel que ceux utilisés dans les implants cardiaques.

Le WO 2008/010220 décrit une autre technique encore, qui est un perfectionnement de la précédente. Le processeur neuronal *spike* y est combiné à un algorithme d'apprentissage renforcé *(Q-learning),* qui apprend et associe les conditions cardiaques aux délais optimaux. L'utilisation de cet apprentissage renforcé permet de renforcer l'immunité aux bruits et d'augmenter la vitesse de convergence de l'algorithme d'asservissement. Toutefois, s'il permet d'atteindre d'excellentes performances, cet apprentissage renforcé nécessite des ressources matérielles dédiées supplémentaires, notamment l'ajout d'un microcontrôleur en plus du processeur neuronal *spike.*

L'invention a pour but de remédier aux inconvénients exposés ci-dessus, en proposant une nouvelle technique simple, rapide, automatisée et efficace permettant d'optimiser concomitamment les deux paramètres DAV et DVV, et ceci malgré le caractère interdépendant de ces deux paramètres.

On verra en particulier que la technique de l'invention peut être appliquée quasiment en temps réel, c'est-à-dire avec un temps de réponse de quelques cycles cardiaques seulement, et en ne mettant en oeuvre que des moyens matériels et logiciels simples, tout à fait compatibles avec ce qui est disponible au sein d'un implant cardiaque actuel.

Le point de départ de l'invention consiste, pour optimiser le couple de paramètres {DAV, DVV}, à exploiter non plus deux caractéristiques distinctes (c'est-à-dire des représentations unidimensionnelles de deux fonctions monovariable distinctes), mais une surface hémodynamique, c'est-à-dire une fonction Z à deux variables Z = f(DAV, DVV).

Cette surface hémodynamique caractérise le système formé par l'ensemble : dispositif CRT + coeur du patient + capteur hémodynamique, et représente une fonction qui varie en fonction des DAV et DVV programmés dans le dispositif CRT, ainsi que de l'état du patient et du capteur utilisé. L'invention propose également d'appliquer la théorie de l'asservissement des systèmes discrets, en utilisant pour assurer le suivi ou *tracking* du point hémodynamique optimal sur la surface hémodynamique un contrôleur numérique de type PID conventionnel, ne nécessitant donc que des moyens matériels et/ou logiciels limités.

Plus précisément, l'invention propose un dispositif médical actif, comprenant un dispositif médical de type connu d'après le US 6 792 310 B1 précité, comprenant les éléments énoncés dans la partie caractéristique de la revendication 1.

De façon caractéristique de l'invention, le dispositif comprend les éléments énoncés par la partie caractérisante de la revendication 1. Les sous-revendications visent des formes de réalisation subsidiaires avantageuses.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La Figure 1 est un synoptique montrant les divers éléments du système impliqués dans l'asservissement hémodynamique temps réel du dispositif CRT selon l'invention.

La Figure 2 est une représentation d'une surface hémodynamique, dans le cas d'un capteur mesurant les différences entre pression systolique et pression diastolique.

La Figure 3 est une représentation d'une surface hémodynamique, dans le cas d'un capteur mesurant la valeur du pic d'accélération endocardiaque PEA.

La Figure 4 illustre le schéma de base d'une boucle à contrôleur PID appliquée à l'asservissement hémodynamique d'un dispositif CRT.

La Figure 5 illustre le cas d'une optimisation séparée des DAV et DVV indépendamment, sans interaction.

La Figure 6 illustre la variation de la fonction hémodynamique en fonction du DAV ou du DVV, dans le cas d'un capteur mesurant les différences entre pression systolique et pression diastolique.

La Figure 7 illustre la variation de la fonction hémodynamique en fonction du DAV, dans le cas d'un capteur mesurant la valeur du pic d'accélération endocardiaque PEA.

La Figure 8 montre la manière d'obtenir l'optimisation du paramètre hémodynamique au moyen de deux boucles PID interdépendantes avec modulation/démodulation du DAV et du DVV.

La Figure 9 est un chronogramme montrant la manière dont évoluent les paramètres DAV et DVV pendant la recherche de l'optimum, au fil des cycles cardiaques successifs.

La Figure 10 illustre la réponse de l'asservissement PID à chaque pas d'adaptation, pour divers paramètres de gain de la boucle.

La Figure 11 illustre différentes caractéristiques possibles du signal de capteur en fonction du délai (DAV ou DVV), pour l'obtention d'un signal d'erreur de pilotage de l'asservissement.

On va maintenant décrire des exemples de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux de la famille *Paradym CRT* produits et commercialisés par Sorin CRM, Montrouge, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail. On a illustré sur la Figure 1 de façon schématique, sous forme de blocs fonctionnels, les différents éléments impliqués dans la technique d'asservissement hémodynamique temps réel des paramètres DAV et DVV selon l'invention.

La référence 10 désigne le générateur du dispositif CRT, qui est interfacé avec le coeur au moyen de sondes permettant de détecter les signaux de dépolarisation du myocarde et stimuler celui-ci par délivrance d'impulsions aux différentes cavités : une sonde 12 implantée dans l'oreillette droite (AR) et une sonde 14 implantée dans le ventricule droit (VR) permettent d'optimiser le délai atrioventriculaire DAV entre les instants de stimulation de l'oreillette et du ventricule droit, tandis qu'une sonde 16 implantée au voisinage du ventricule gauche (VL) permet, en combinaison avec la sonde 14 implantée dans le ventricule droit, d'optimiser le délai interventriculaire DVV entre les ventricules gauche et droit.

Par ailleurs, un capteur hémodynamique 18 mesure une quantité corrélée avec le débit cardiaque.

Les capteurs hémodynamiques sont des capteurs permettant d'estimer les variations de contractilité, corrélées aux augmentations de la pression sanguine. Ils se distinguent des capteurs d'activité (capteurs d'accélération par exemple) et des capteurs métaboliques (capteurs de ventilation-minute par exemple), qui ne sont destinés qu'à diagnostiquer la présence ou non d'un exercice par le patient et à quantifier ses besoins métaboliques, par exemple pour adapter la fréquence cardiaque de stimulation en fonction du niveau détecté. Les capteurs hémodynamiques peuvent en revanche non seulement assurer un suivi des efforts du patient comme les capteurs d'activité et métaboliques, mais également donner une indication de la tolérance hémodynamique du patient par rapport à certains événements, en particulier la tolérance à une modification des paramètres DAV et DVV par le dispositif.

Dans les exemples que l'on décrira ici, on prendra pour exemple de capteur hémodynamique soit un capteur de pression intracardiaque permettant de déterminer la différence de pression $\Delta pp$ entre la pression systolique et la pression diastolique, soit un capteur endocavitaire d'accélération endocardiaque de type capteur PEA *(Peak Endocardial Acceleration).* Ces exemples ne sont toutefois aucunement limitatifs, et l'invention peut être mise en oeuvre avec d'autres capteurs hémodynamiques tels que : capteur d'accélération épicardique (et non plus endocavitaire), capteur de mouvement d'une paroi du myocarde, capteur de bioimpédance intracardiaque, capteur de saturation d'oxygène par mesure optique, capteur de mesure de variation de volume par ultrasons, etc.

L'invention peut être également mise en oeuvre dans une configuration où l'analyse est opérée à partir d'un signal obtenu de manière non invasive, au moyen d'un capteur externe au lieu d'un capteur implanté, par exemple au moyen d'un capteur accélérométrique fixé sur la poitrine du patient au niveau du sternum.

Pour diverses descriptions de capteurs hémodynamiques, on pourra se référer notamment aux documents suivants :

- pour un capteur d'accélération endocardiaque de type PEA : le EP 0 515 319 A1 (Sorin Biomedica Cardio SpA), qui décrit la manière de recueillir un signal d'accélération endocardiaque au moyen d'une sonde endocavitaire pourvue d'une électrode distale de stimulation implantée au fond du ventricule et intégrant un micro-accéléromètre permettant de mesurer l'accélération endocardiaque, et le EP 0 655 260 A1 (Sorin Biomedica Cardio SpA), qui décrit une manière de traiter le signal d'accélération endocardiaque mesuré pour en dériver notamment la valeur des pics d'accélération endocardiaque correspondant aux deux bruits majeurs qu'il est possible de reconnaître dans chaque cycle d'un coeur sain ;

- pour un capteur de bioimpédance transvalvulaire (entre oreillette et ventricule situés d'un même côté du coeur) : le EP 1 116 497 A1 (ELA Medical), qui décrit une mesure dynamique de la bioimpédance (BioZ) afin d'évaluer les volumes diastolique et systolique et obtenir ainsi une indication du débit cardiaque et donc de la fraction d'éjection ;

- pour un capteur de bioimpédance transseptale (entre un site situé d'un côté du coeur et un site situé de l'autre côté) : le EP 1 138 346 A1 (ELA Medical), qui décrit un autre type de mesure permettant de délivrer également une valeur représentative de la fraction d'éjection ;

- pour un capteur accélérométrique externe : le EP 1 741 387 A1 (ELA Medical), qui décrit la manière de recueillir un signal d'accélération endocardiaque au moyen d'une sonde placée sur le thorax du patient.

Qu'il s'agisse d'un capteur implanté (endocavitaire, épicardique, ... ) ou externe, celui-ci délivre un signal corrélé avec le débit cardiaque qui est transmis à un circuit d'acquisition des mesures 20 (ce circuit pouvant être incorporé au générateur du dispositif implanté, ou bien situé à l'extérieur du corps du patient).

Le circuit 20 délivre un signal Z, ci-après "signal hémodynamique", qui est transmis à une unité 22 formant contrôleur d'asservissement. Cette transmission peut être directe (dans le cas d'un traitement purement interne à l'implant), ou bien être opérée par télémétrie (dans le cas d'un capteur externe et d'un contrôleur 22 intégré à l'implant ou, inversement, dans le cas d'un capteur implanté et d'un contrôleur 22 externe, par exemple un contrôleur intégré à un programmateur utilisé pour paramétrer le générateur lors d'une visite chez un praticien).

Le contrôleur 22 met en oeuvre un algorithme d'asservissement permettant d'obtenir concurremment les valeurs optimales recherchées du DAV et du DVV.

On notera qu'il est possible d'utiliser plusieurs capteurs pour asservir un même délai (par exemple un accéléromètre et un capteur de bioimpédance pour asservir le DAV), ou encore d'utiliser un capteur différent pour chacun des délais (par exemple un capteur de bioimpédance pour asservir le DVV et un capteur PEA pour asservir le DAV).

En tout état de cause, quel que soit le type de capteur(s) utilisé(s), le bloc 20 délivre une information Z qui est une fonction à deux variables Z = f(DAV, DVV) qui peut être représentée graphiquement par une surface appelée ci-après "surface hémodynamique".

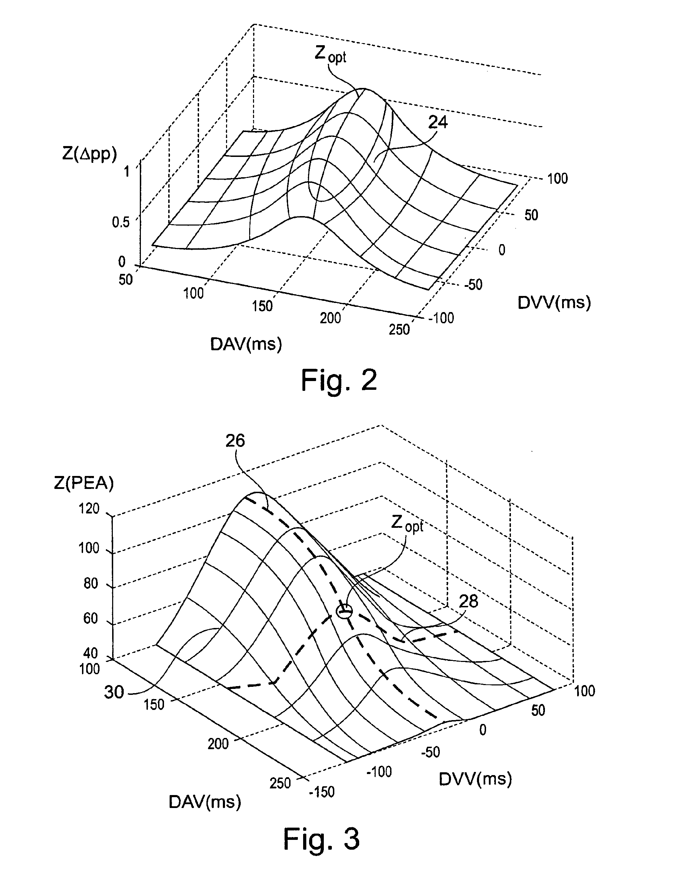

La Figure 2 illustre un exemple d'une telle surface hémodynamique Z = f(DAV, DVV) dans le cas où le capteur utilisé est un capteur de pression sanguine permettant d'obtenir une information Z = $\Delta$pp représentative d'une différence entre la pression systolique et la pression diastolique. L'optimum de cette surface 24 est situé au point $Z_{opt}$ présentant la cote Z la plus élevée. En pratique, au voisinage de cet optimum, le sommet de la surface hémodynamique 24 peut être approximé par des caractéristiques paraboliques, dans le plan {DVV, Z} et dans le plan {DAV, Z}.

La Figure 3 illustre un autre exemple de surface hémodynamique Z = f(DAV, DVV), dans le cas où le capteur est un capteur d'accélération endocardiaque délivrant un signal permettant d'obtenir une information Z représentative du pic d'accélération endocardiaque PEA. Plus précisément, le premier pic d'accélération endocardiaque ("PEA1") correspond à la fermeture des valvules mitrale et tricuspide, au début de la phase de contraction ventriculaire isovolumique (systole), et ses variations sont étroitement liés aux variations de la pression dans le ventricule et peuvent donc constituer un paramètre représentatif de la contractilité du myocarde. L'amplitude du pic PEA1 est notamment corrélée au maximum positif de la variation de pression dP/dt dans le ventricule gauche.

Les variations du signal en fonction du DAV suivent une loi Z = f(DAV) représentée par une caractéristique 26 en forme de sigmoïde, tandis que les variations de ce même signal en fonction du DVV suivent une loi Z = f(DVV) représentée par une caractéristique 28 approximativement parabolique.

Dans ce cas, la valeur optimale du couple {DAV, DVV} correspond au point $Z_{opt}$, situé, pour le DAV, au point d'inflexion de la caractéristique sigmoïde 26 et, pour le DVV, au sommet de la caractéristique parabolique 28.

La Figure 4 illustre sous forme fonctionnelle le schéma de base d'une boucle d'asservissement du système de la Figure 1.

Il s'agit de réaliser un contrôleur 22 générant en sortie les deux valeurs DAV et DVV, à partir d'une entrée $E_z$ représentant le signal d'erreur produit en combinant en 32 d'une part la valeur courante Z = f(DAV, DVV) obtenue à partir de la valeur mesurée par le capteur hémodynamique, et d'autre part une valeur de consigne correspondant à l'optimum recherché $Z_{opt}$ de la surface hémodynamique 30, surface qui caractérise la réponse Z = f(DAV, DVV) de l'ensemble constitué par : le générateur de resynchronisation CRT 10, le coeur du patient, le capteur hémodynamique 18 et sa chaîne d'acquisition 20.

L'invention propose d'utiliser pour l'unité 22 un contrôleur numérique tel qu'un contrôleur PID, qui est un organe de contrôle pour régulation en boucle fermée délivrant en sortie un signal de commande :

- proportionnel ("P") au signal d'erreur appliqué en entrée ;
- calculé en intégrant ("1") cette erreur, c'est-à-dire que la commande délivrée en sortie augmentera en permanence si l'erreur est constante (en espérant ainsi que l'erreur finira par décroître) ; et
- proportionnel à la dérivée ("D") de l'erreur, pour créer une accélération de la correction dans le cas où l'erreur s'accroîtrait brutalement.

Dans le cas d'un contrôleur numérique opérant sur des valeurs discrètes, un tel asservissement fonctionne de façon discontinue, par pas successifs (chaque pas correspondant dans le cas présent à un cycle cardiaque), dans la mesure où il est nécessaire d'attendre la fin du cycle k pour mesurer la valeur $Z_k$ qui représente la valeur de la fonction hémodynamique pendant ce cycle.

Un contrôleur PID conventionnel ne peut toutefois pas être utilisé directement pour mettre en oeuvre l'invention, et ce pour deux raisons :

- la valeur de consigne $Z_{oPt}$ à suivre n'est pas connue ou pas accessible a *priori* ; et
- un contrôleur PID classique est un filtre à une entrée et une sortie, alors que dans le cas présent il est nécessaire de suivre simultanément deux variables, à savoir le DAV et le DVV.

Il n'est pas non plus possible de dédoubler le système de manière à asservir séparément le DAV et le DVV simultanément au moyen de deux contrôleurs PID, car le dédoublement des contrôleurs n'assurerait pas la convergence vers le point optimal.

Cet inconvénient est expliqué plus précisément en référence à la Figure 5. Sur cette figure, on a pris l'exemple d'une surface hémodynamique correspondant à la Figure 2, où le paramètre Z est la variation de pression $\Delta pp$ : dans ce cas, dans un plan {DAV,DVV} les courbes de niveau correspondent approximativement à des ellipses, représentées en pointillés sur la Figure 5, avec un optimum global $Z_{oPt}$ au point P.

Si l'on fixe la valeur du DAV à DAV1 et que l'on effectue un balayage du DVV à DAV constant selon S1, alors le maximum trouvé sera DVV = DVV1. Si à partir de ce maximum DVV1 on effectue un balayage du DAV à DVV constant selon S2, alors on trouvera un maximum au point P1.

Si, en revanche, on fixe la valeur du DVV à DVV2 et que l'on effectue un balayage du DAV à DVV constant selon S3, on trouve un maximum local DAV = DAV2. Si à partir de ce maximum DAV2 on effectue un balayage du DVV à DAV constant selon S4, on trouvera alors un maximum au point P2.

Ainsi, une optimisation du DVV puis du DAV conduit à un optimum apparent au point P1, tandis qu'une optimisation du DAV puis du DVV conduit à un optimum apparent au point P2, ces deux points P1 et P2 étant non seulement différents l'un de l'autre, mais en outre différents de l'optimum réel, qui se trouve au point P.

Un algorithme mettant en oeuvre deux asservissements indépendants, l'un pour le DAV et l'autre pour le DVV, ne permettrait donc pas d'atteindre l'optimum global recherché.

On va maintenant décrire la manière dont opère l'algorithme de l'invention, qui prévoit, de façon caractéristique :

- en premier lieu, de générer un signal d'erreur destiner à guider le contrôleur numérique PID vers le point optimal $Z_{opt}$, et
- en second lieu, l'utilisation du signal d'erreur ainsi généré pour asservir simultanément les délais AV et VV.

*Génération du signal d'erreur*

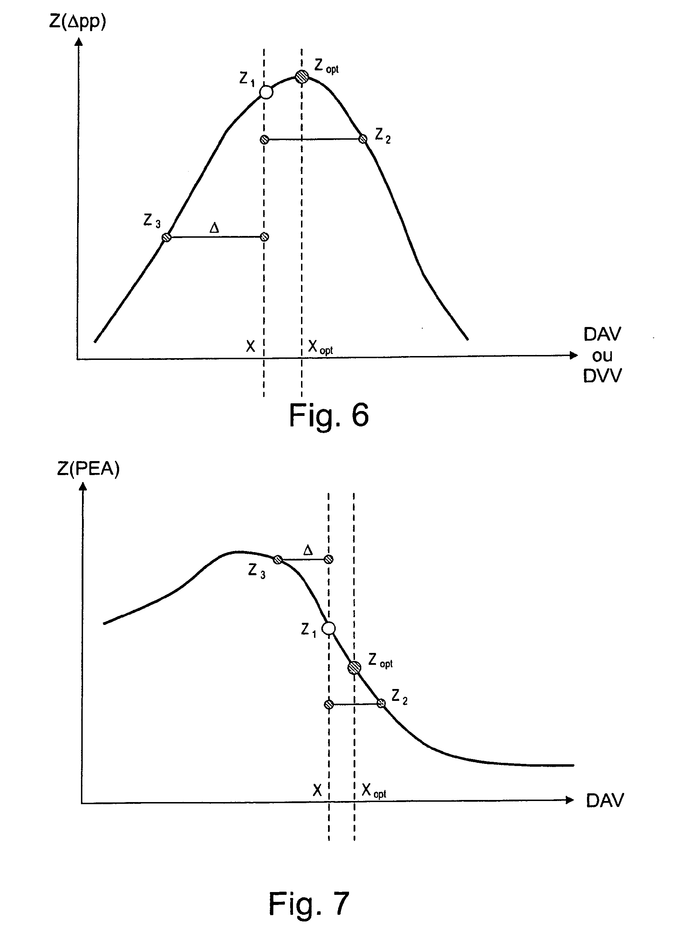

On va tout d'abord décrire la manière de générer le signal d'erreur, en référence aux Figures 6 et 7.

Afin de connaître la position des valeurs de DAV et de DVV courantes (c'est-à-dire des valeurs actuellement programmées dans le dispositif) par rapport aux valeurs optimales $DAV_{opt}$ et $DVV_{oPt}$ correspondant à l'optimum global $Z_{opt}$, on va :

- réaliser une modulation de ces délais, c'est-à-dire que l'on va faire varier d'une façon déterministe et connue les délais autour de leur valeur courante, et
- observer les variations résultantes du signal hémodynamique Z pour déduire un signal d'erreur, l'extraction de l'information concernant le signal d'erreur se faisant par une démodulation.

La modulation et la démodulation s'appliquent séparément au DAV et au DVV, ou bien en même temps sur les deux délais. Pour garantir un confort du patient on préférera toutefois une modulation du DAV indépendante de la modulation du DVV.

D'autre part, le choix de la modulation et de la démodulation dépend de la forme de la surface hémodynamique.

Sur la Figure 6, on a illustré le cas d'un capteur de pression, permettant d'évaluer comme paramètre hémodynamique Z la différence $\Delta pp$ entre la pression systolique et la pression diastolique.

Au voisinage de l'optimum $Z_{opt}$, la surface hémodynamique est une surface approximativement parabolique, aussi bien pour une variation du DAV que pour une variation du DVV. On notera X indifféremment la valeur du délai DAV ou du délai DVV.

On supposera que la fonction $Z = H(X-X_{opt})$ est une fonction paire autour de $X_{opt}$. Concrètement, toute fonction semblable possédant un maximum peut être approchée par une fonction quadratique symétrique dans un voisinage de $X_{opt}$) et, en tout état de cause, si la fonction n'est pas parfaitement symétrique l'algorithme d'asservissement convergera vers un délai $X'_{opt}$ très proche de $X_{opt}$, la différence n'ayant pas de conséquence sensible sur le résultat final du point de vue clinique pour le patient.

La modulation du délai X consiste à faire varier ce délai autour de sa valeur courante, avec un pas de modulation de $\Delta$ millisecondes. Le dispositif sera ainsi programmé séquentiellement avec trois valeurs différentes de DAV (ou de DVV), selon le cas :

- X, la valeur courante du DAV (ou du DVV), qui donne une valeur hémodynamique $Z_1 = H(X-X_{opt})$ ;
- $X+\Delta$, qui donne une valeur hémodynamique $Z_2 = H(X+4-X_{oPt})$ ; et
- $X-\Delta$, qui donne une valeur hémodynamique $Z_3 = H(X-4-X_{oPt})$.

La démodulation est opérée en multipliant le signal Z mesuré par +1 ou -1, selon le sens de la modulation. En d'autres termes, la démodulation consiste à faire passer les échantillons $Z_2$ et $Z_3$ dans un filtre dérivateur du premier ordre à deux coefficients, +1 et -1 : ainsi, $Z_2$ est multiplié par +1 et $Z_3$ par -1. Le signal d'erreur obtenu à la sortie du démodulateur est donc $E = Z_2-Z_3$.

On démontre que, dans le cas d'une fonction paire H, le signal d'erreur peut s'écrire $E = 2p(X_{oPt}-X)$, $p = -H'(\Delta) = H'(-\Delta)$ étant la pente de la courbe hémodynamique pour $X= X_{opt}-\Delta$. Ainsi le signal d'erreur obtenu après modulation et démodulation est égal, à un gain près, à la différence entre le délai de consigne à suivre $X_{opt}$ et le délai à commander X.

La sensibilité du signal d'erreur dépend ainsi de la pente $p$ de la courbe hémodynamique.

La pente de cette courbe est nulle quand $X = X_{opt}$. Quand le pas $\Delta$ de modulation est très faible, la pente $p$ est aussi faible. Le choix du pas dépend de la qualité du signal hémodynamique.

Quand la fonction hémodynamique est presque plate, la pente p est faible et le signal d'erreur E est peu sensible aux variations de X. On peut détecter ce cas de figure en estimant la courbure de la fonction hémodynamique en utilisant la mesure $Z_1$ non utilisée jusqu'à présent.

La courbure C s'estime par : $C = 2Z_1-Z_2-Z_3$. Quand cette courbure C est inférieure (en valeur absolue) à un seuil prédéfini, il est préférable de ne pas utiliser le signal d'erreur E pour adapter le délai X.

Dans certains cas, l'appareil CRT ne permet de programmer les délais qu'avec un nombre limité de valeurs prédéfinies, et la modulation ne peut donc pas être symétrique autour du délai en cours X. Soient $\Delta_2$ le pas de modulation associé à la mesure $Z_2$ et $\Delta_3$ le pas de modulation associé à la mesure $Z_3$. On démontre alors que le signal d'erreur :

$$E' = 2\frac{(\Delta_2 - \Delta_3)Z_1 + \Delta_3 Z_2 - \Delta_2 Z_3}{(\Delta_2 + \Delta_3)}$$

s'annule quand X coïncide avec $X_{opt}$.

Sur la Figure 7, on a illustré le cas d'un capteur de type accéléromètre, permettant d'évaluer comme paramètre hémodynamique une valeur Z reflétant l'amplitude du PEA en fonction de la variation du DAV.

La caractéristique correspondante présente une forme sigmoïde, et l'optimum $Z_{opt}$ ne correspond pas à un extremum (comme dans le cas de la Figure 6) mais au point d'inflexion de la sigmoïde. La manière de procéder est modifiée de la manière suivante.

On supposera qu'au voisinage de l'optimum la fonction Z peut s'exprimer sous la forme $Z = Z_{opt}+G(X-X_{opt})$, G étant une fonction impaire autour de $X_{opt}$, et X étant la valeur courante du DAV.

De façon générale, toute fonction possédant un point d'inflexion peut être approchée par une fonction impaire dans un voisinage de $X_{opt}$. Si la fonction n'est pas parfaitement impaire l'algorithme d'asservissement convergera vers un délai $X'_{opt}$ très proche de $X_{opt}$, la différence n'ayant pas de conséquence sensible sur le résultat final du point de vue clinique pour le patient.

La modulation consiste, ici encore, à faire varier X autour de sa valeur courante avec un pas de modulation de $\Delta$ millisecondes. Le dispositif sera programmé séquentiellement avec trois valeurs différentes de DAV :

- X, la valeur courante du DAV, qui donne une valeur hémodynamique $Z_1 = z_{opt} + G(X-X_{opt})$ ;
- $X+\Delta$, qui donne une valeur hémodynamique $Z_2 = Z_{opt}+G(X+o-X_{opt})$ ; et
- $X-\Delta$, qui donne une valeur hémodynamique $Z_3 = Z_{opt} +G(X-\Delta-X_{opt})$.

La démodulation consiste à faire passer les échantillons $Z_3$, $Z_1$ et $Z_2$ dans un filtre dérivateur du second ordre à trois coefficients, respectivement -1, +2 et -1. En d'autres termes, la démodulation consiste à multiplier $Z_1$ par 2, $Z_2$ par -1 et $Z_3$ par -1.

Le signal d'erreur obtenu en sortie du démodulateur s'écrit :$E = 2Z_1-Z_2-Z_3$, et l'on démontre que ce signal d'erreur peut s'écrire sous la forme : $E = 2(p_0-p_j)(X_{opt}-X)$, les valeurs $p_0$ et $p_1$ étant celles des pentes (positives) $P_1 = -G'(\Delta)$ et $p_0 = -G'(0)$.

Ainsi, le signal d'erreur obtenu après une modulation et une démodulation est égal, à un gain près, à la différence entre le délai de consigne à suivre $X_{opt}$ et le délai à commander X.

Lorsque Z varie linéairement en fonction de X, tout point X donne un signal d'erreur nul car la dérivée seconde (qui définit le point d'inflexion) est partout nulle, avec $p_0 = p_1$.

La sensibilité du signal d'erreur dans le cas de la recherche d'un point d'inflexion dépend donc de la différence $p_0-p_1$, de sorte qu'il vaut mieux choisir un pas de modulation $\Delta$ assez grand pour maximiser cette sensibilité, en évitant des pentes $p_0$ et $p_1$ trop proches. Quand la fonction hémodynamique est presque plate, la différence $p_0-p_1$ est petite, et le signal d'erreur E est peu sensible aux variations de X. Dans ce cas, il est préférable de ne pas utiliser le signal d'erreur E pour adapter le délai X. D'autre part, la plupart des dispositifs CRT ne permettent généralement de programmer les délais (DAV ou DVV) qu'avec un nombre limité de valeurs prédéfinies, de sorte que la modulation ne pourra pas être parfaitement symétrique autour du délai courant X. Soient $\Delta_2$ le pas de modulation associé à la mesure $Z_2$ et $\Delta_3$ le pas de modulation associé à la mesure $Z_3$. On démontre alors que le signal d'erreur :

$$E' = 2\frac{(\Delta_2 + \Delta_3)Z_1 - \Delta_3 Z_2 - \Delta_2 Z_3}{(\Delta_2 + \Delta_3)}$$

s'annule quand X coïncide avec $X_{opt}$.

La séquence de modulation/démodulation au cours des cycles cardiaques peut être opérée de la manière suivante (indifféremment pour le DAV ou pour le DVV) :

> a) mesure du signal hémodynamique $Z_1$, pour une valeur X du DAV ou du DVV,
> b) modification du délai : X = X+$\Delta$,
> c) mesure du signal hémodynamique $Z_2$ résultant,
> d) modification du délai X = X-$\Delta$,
> e) mesure du signal hémodynamique $Z_3$ résultant,
> f) calcul du signal d'erreur E = $Z_2$-$Z_3$ (ou E = 2$E_1$,-$Z_2$-$Z_3$, selon le cas).

On notera que la mesure des signaux $Z_1$, $Z_2$ et $Z_3$ aux étapes a), c) et e) peut se faire soit sur l'intervalle d'un seul cycle cardiaque, soit bien en prenant la valeur moyenne de mesures opérées sur plusieurs cycles cardiaques, le choix étant en fait fonction de la rapidité de la réponse du paramètre mesuré aux variations du DAV ou du DVV. D'autre part, pour certains capteurs hémodynamiques, il peut être nécessaire d'attendre un ou plusieurs cycles cardiaques pour que la réponse hémodynamique se stabilise après modification du délai aux étapes b) et d).

*Asservissement simultané du DA V et du DVV à partir du signal d'erreur*

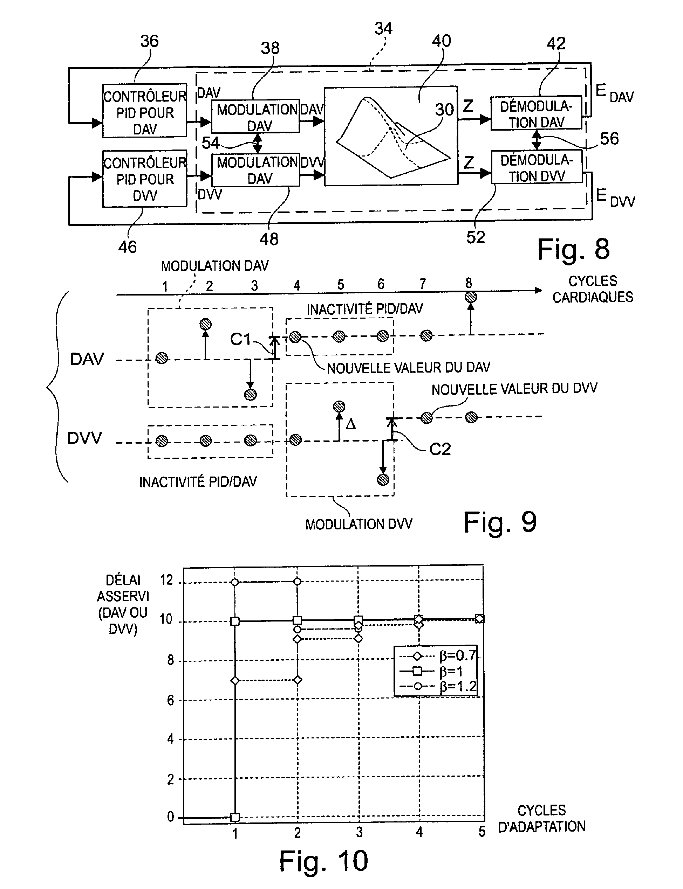

On va maintenant décrire, en référence aux Figures 8 à 10, la manière dont l'algorithme utilise les signaux d'erreur de DAV et au DVV générés par la technique de modulation/démodulation que l'on vient d'exposer ci-dessus.

Il est en particulier possible d'utiliser la théorie classique des systèmes asservis pour réaliser un contrôleur numérique simple permettant d'assurer le suivi des DAV et DVV optimaux.

Comme illustré Figure 8, le système comprend deux boucles d'asservissement correspondant à chacun des deux délais DAV et DVV. Chacune de ces boucles comprend un contrôleur dédié respectif 36, 46, notamment un contrôleur de type PID (type non limitatif), associé au bloc de génération d'erreur 34.

La boucle d'asservissement du DAV comprend un contrôleur PID 36 recevant en entrée un signal d'erreur $E_z$ propre au DAV et délivrant en sortie un signal de commande de DAV appliqué à un circuit modulateur 38 permettant, comme exposé plus haut en référence à la Figure 6, de moduler de façon contrôlée la valeur du DAV autour de la valeur optimale recherchée.

Le bloc 40 illustre la réponse globale à une variation du DAV du système comprenant (cf. Figure 1) le générateur CRT 10, le coeur, le capteur hémodynamique 18 et le circuit d'acquisition des mesures 20. Ce bloc 40, qui incorpore donc la fonction représentée par la surface hémodynamique 30, donne une valeur de signal hémodynamique Z qui est démodulée par le bloc 42, pour donner le signal d'erreur $E_z$ appliqué en entrée du contrôleur PID 36.

De façon semblable, la boucle d'asservissement du DVV comprend un contrôleur PID 46 recevant en entrée un signal d'erreur $E_z$ propre au DVV et délivrant en sortie un signal de commande de DVV appliqué à un circuit modulateur 48 permettant, comme exposé plus haut en référence aux Figures 6 et 7, de moduler de façon contrôlée la valeur du DVV autour de la valeur optimale recherchée. Le bloc 40 donne une valeur de signal hémodynamique Z qui est démodulée par le bloc 52, pour donner le signal d'erreur $E_z$ appliqué en entrée du contrôleur PID 46.

Comme on l'a expliqué plus haut en référence à la Figure 5, une recherche indépendante des optima pour le DAV et le DVV conduit généralement à des optima locaux, différents de l'optimum global recherché.

Il est donc nécessaire de rendre interdépendante l'action des deux boucles d'asservissement, par une interaction entre les blocs de modulation et de démodulation des DAV et DVV, comme illustré par les flèches 54 et 56.

Cette interaction peut notamment résulter d'un séquencement spécifique des cycles de modulation/démodulation du DAV et du DVV, respectivement.

Un exemple de ce séquencement spécifique est illustré Figure 9.

Comme on l'a indiqué plus haut (cf. les explications relatives à la génération du signal d'erreur), le signal d'erreur E est disponible après démodulation des valeurs $Z_1$, $Z_2$ et $Z_3$, correspondant aux valeurs successives respectives X, X+$\Delta$ et X-$\Delta$ du délai X (DAV ou DVV).

Il faut donc ainsi au moins trois cycles cardiaques pour obtenir un signal d'erreur pour chacun des délais DAV ou DVV.

Le contrôleur PID 36 (ou 46), qui fonctionne de manière séquentielle, opère une mise à jour du délai qu'il pilote après multiplication de ce signal d'erreur par un gain, puis intégration.

Pour le DAV, le contrôleur PID correspondant 36 attend trois cycles cardiaques pour recevoir un signal d'erreur. Il effectue alors une mise à jour du DAV commandé en sortie, et reste inactif pendant les trois cycles cardiaques suivants, au cours desquels le contrôleur PID du DVV 46 prend la main, et ainsi de suite, le fonctionnement alterné des deux contrôleurs PID 36 et 46 permettant d'assurer la convergence et le suivi simultané des deux délais optimaux $DAV_{oPt}$ et $DVV_{oPt}$.

Sur la Figure 9, on a illustré les valeurs des délais DAV et DVV pendant une séquence de huit cycles cardiaques.

Pendant les cycles n°1 à n°3, le DAV est modulé et le DVV est maintenu à sa valeur courante (inhibition de la modulation du DVV). A la fin du cycle n°3, un signal d'erreur C1 est calculé pour le DAV. Ce signal C1 est appliqué en entrée du contrôleur PID 36 dédié au DAV, et une nouvelle valeur du DAV est ainsi déterminée, obtenue en sortie de ce contrôleur.

Le DVV est alors modulé pendant les cycles n°4 à n°6, en maintenant inchangée la valeur du DAV que l'on vient de calculer (c'est-à-dire que la modulation du DAV est inhibée pendant ces trois cycles). A la fin du cycle n°6 un signal d'erreur C2 du DVV est calculé et appliqué au contrôleur PID 46 dédié au DVV, donnant en sortie une nouvelle valeur du DVV appliquée au dispositif (cycle n°7). Au cycle n°8 le processus que l'on vient de décrire est réitéré en appliquant une modulation DAV, et ainsi de suite, les cycles n°7, 8 et 9 correspondant aux cycles n°1, 2 et 3 de l'itération précédente.

Un cycle complet d'adaptation du système à des nouvelles valeurs du couple DAV/DVV nécessite ainsi six cycles cardiaques au moins.

On va maintenant évaluer les conditions de stabilité et de convergence du système à double boucle que l'on vient de décrire.

Soit $k$ l'indice de temps pour un contrôleur PID (le contrôleur 36 pour le DAV ou le contrôleur 46 pour le DVV). Un cycle d'adaptation du système asservi correspond au passage de $k$ à $k+1$, c'est-à-dire à une durée de six cycles cardiaques au moins.

Le signal d'erreur $E_k$ fourni au contrôleur PID s'écrit :

$$E_k = s\left(X_{opt} - X_k\right)$$

$s$ étant la sensibilité de chaque contrôleur, valeur qui dépend du pas de modulation $\Delta$ ainsi que de la forme de la fonction hémodynamique.

Le contrôleur PID corrige $X_k$ selon la relation :

$$X_{k+1} = X_k + gE_k$$

$g$ étant le gain de la boucle.

La transformée en z de la fonction de transfert $K$ entre le délai asservi X courant et le délai optimum recherché $X_opt$ s'écrit alors :

$$\frac{X}{X_{opt}} = K(z) = \frac{\beta}{z - 1 + \beta}$$

où $\beta = g.s.$

Le comportement de la boucle d'asservissement à long terme se détermine par la limite de cette fonction quand z tend vers 1. Comme $\lim_{z \to 1} K(z) = 1$ et que la boucle est fermée, le délai asservi $X$ converge vers $X_{opt}$ à condition que la condition de stabilité de cette boucle soit vérifiée.

Cette condition peut être exprimée sous la forme :

$$0 < \beta < 2 \Leftrightarrow 0 < g < \frac{2}{s}$$

Le gain de la boucle dépend ainsi de la sensibilité du bloc générateur d'erreur.

La figure 10 montre l'évolution du délai asservi X en réponse à un échelon dans $X_{opt}$, de 10 ms pour trois valeurs de $\beta$ : on peut remarquer que pour $\beta = 1$ le délai asservi coïncide avec le délai optimum au bout d'un seul cycle d'adaptation, tandis que pour $\beta < 1$ le système converge d'une façon monotone et que pour $\beta > 1$ le délai asservi oscille autour de $X_opt$.

En pratique, la valeur de la sensibilité s n'est pas connue. Elle peut toutefois être estimée pour un patient donné et un capteur donné, une fois au moins, par exemple au moment de l'implantation du capteur. En tout état de cause, il est néanmoins préférable de choisir un gain inférieur à 1/s, pour éviter les oscillations et laisser une marge de stabilité si la sensibilité s augmente au cours du temps.

Une autre façon d'assurer la stabilité consiste à appliquer un algorithme d'asservissement de type "PID tronqué", c'est-à-dire avec limitation des corrections $c_k$ = min(20,max( -20, $gE_k$)) à une valeur maximale donnée, par exemple des corrections limitées à $\pm$ 20 ms.

Un exemple détaillé d'un tel algorithme de type "PID tronqué", appliqué au cas d'un signal hémodynamique délivré par un capteur PEA est donné en ANNEXE 1 ci-après.

Dans une variante de mise en oeuvre de l'algorithme, on peut appliquer un algorithme d'asservissement de type "PID signe", avec un pas d'adaptation fixe, de 5 ms par exemple. Dans un tel cas chaque contrôleur PID effectue une mise à jour selon la relation $X_{k+1}$, $= X_k$, $+ 5. signe(E_k)$, dans laquelle *signe(...)* représente la fonction signe (égale à 1 si $E_k$ est positif, et à -1 sinon).

Les étapes 6 à 9 de l'algorithme de l'ANNEXE 1 ci-après deviennent alors :

     1. E= 2*Z1-Z2-Z3
     2. AVp= AVp+ signe(E)*5

Cette variante a l'avantage d'être très robuste, mais présente en revanche l'inconvénient d'osciller perpétuellement autour du délai optimum.

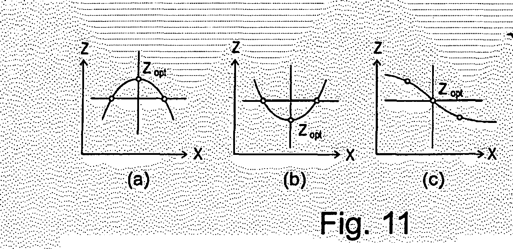

Sur la Figure 11 on a illustré plusieurs types de caractéristiques montrant le signal Z délivré par le capteur en fonction de la variation du délai X (DAV ou DVV).

La Figure 11(a) correspond au cas illustré Figure 6, où la caractéristique est telle que l'optimum $Z_{opt}$ soit situé au maximum de la courbe (supposée paire en ce point), cet optimum pouvant être atteint par approximations successives au moyen de la technique de modulation/démodulation décrite plus haut. Cette caractéristique est typiquement celle délivrée par un capteur donnant la différence $\Delta pp$ entre pression systolique et pression diastolique.

La Figure 11(b) correspond à une caractéristique du même type, pour laquelle il convient de rechercher non plus un maximum mais un minimum de la courbe, celle-ci étant, ici encore, supposée paire au voisinage de l'optimum $Z_{opt}$. Cette caractéristique est typiquement obtenue en mesurant la largeur du complexe QRS de l'ECG. On pourra notamment se référer à David Tamborero et al., Optimization of the Interventricular Delay in Cardiac Resynchronization Therapy Using the QRS Width, American Journal of Cardiology, 15 Nov. 2009, Vol. 104, Issue 10, pp. 1407-1412, qui décrit un procédé d'optimisation dans lequel le délai DVV optimum correspond au complexe QRS le moins large.

La Figure 11(c) correspond au cas illustré Figure 7, avec une caractéristique de type sigmoïde, typique notamment d'un signal délivré par un capteur d'accélération endocardiaque, tel qu'un capteur donnant la valeur du pic PEA en fonction du DAV. Il convient dans ce cas de rechercher comme optimum $Z_{opt}$ le point d'inflexion de la courbe, supposé impaire au voisinage de ce point, avec ici encore génération du signal d'erreur par une technique de modulation/démodulation comme exposé plus haut.

ANNEXE 1

*Algorithme d'asservissement de type "PID tronqué"*

a) Effectuer un balayage complet au moment de la pose du capteur
b) Estimer la pente p pour la courbe VV, p0 et p1 pour la courbe AV, la pente minimum Pseuil pour AV et la courbure minimum Cr_seuil pour VV
c) Choisir le gain g1 = 0.35/p pour la boucle VV
d) Choisir le gain g2 = 0.35/(p0-p1) pour la boucle AV
e) AVp= 120 ms, VVp=0 ms, Delta 1 = 10 ms, Delta2 = 15 ms
f) Programmer AV = AVp et VV = VVp
g) Commencer la boucle d'asservissement :

1. Mesure Z1
2. Programmer AV = AVp + Delta2
3. Mesurer Z2
4. Programmer AV = AVp - Delta2
5. Mesurer Z3
6. E= g2*(2*z1-z2-z3)
7. P =Z3- Z2
8. C= min(20,max(-20,E)
9. si (P > Pseuil) AVp= AVp+ C
10. Programmer AV = AVp
11. Mesure Z1
12. Programmer VV = VVp + Delta1
13. Mesurer Z2
14. Programmer VV = VVp - Delta1
15. Mesurer Z3
16. E- g1*(22-23)
17. C= min(20,max(-20,E)
18. Cr = 2*Z1-Z2-Z3
19. si (Abs(Cr) > Cr_seuil) VVp= VVp+ C
20. Programmer VV = VVp
21. Aller à l'étape 1

**Revendications**

1. Un dispositif médical actif, comprenant :

- une prothèse cardiaque implantable de resynchronisation par stimulation biventriculaire, comportant des moyens de détection d'événements auriculaires et ventriculaires et des moyens (10) de stimulation des ventricules droit et gauche ;
- au moins un capteur (18) apte à délivrer un signal (Z) représentatif d'un paramètre hémodynamique courant du patient ;
- des moyens pour appliquer aux moyens de stimulation un délai atrio-ventriculaire DAV, compté à partir de la détection d'un événement auriculaire spontané ou stimulé et à l'issue duquel une stimulation du ventricule droit est appliquée en l'absence d'événement ventriculaire spontané détecté ;
- des moyens pour appliquer aux moyens de stimulation un délai inter-ventriculaire DVV entre les instants respectifs de stimulation des ventricules droit et gauche ; et
- des moyens d'asservissement (22), aptes à contrôler de façon continue le DAV et le DVV en fonction du signal hémodynamique délivré par le capteur, comprenant :

a) des moyens générateurs de signal d'erreur de DAV (38, 42) aptes à générer, en fonction du signal délivré par ledit au moins un capteur (18), un signal d'erreur de DAV ($E_{DAV}$) représentatif d'un écart entre la valeur courante du DAV et une valeur optimale de DAV ($DAV_{opt}$) ;
b) des moyens générateurs de signal d'erreur de DVV (48, 52) aptes à générer, en fonction du signal délivré par ledit au moins un capteur (18), un signal d'erreur de DVV ($E_{DVV}$) représentatif d'un écart entre la valeur courante du DVV et une valeur optimale de DVV ($DVV_{opt}$) ;

c) un régulateur (36) en boucle fermée de contrôle du DAV, recevant en entrée ledit signal d'erreur de DAV ($E_{DAV}$) et délivrant en sortie un signal de DAV ; et

d) un régulateur (46) en boucle fermée de contrôle du DVV, recevant en entrée ledit signal d'erreur de DVV ($E_{DVV}$) et délivrant en sortie un signal de DVV,

dispositif **caractérisé en ce que** :

- les moyens générateurs de signal d'erreur de DAV (38, 42) sont aptes à opérer, sur un intervalle d'au moins trois cycles cardiaques, par :

· application au DAV courant (CYCLE 1) d'un pas de modulation ($\Delta$) en plus et en moins au cours de cycles cardiaques successifs respectifs (CYCLE 2, CYCLE 3), puis

· calcul dudit signal d'erreur de DAV par différence entre les valeurs respectives de signal hémodynamique mesurées avec les pas de modulation en plus et en moins,

- les moyens générateurs de signal d'erreur de DVV (48, 52) sont aptes à opérer, sur un intervalle d'au moins trois cycles cardiaques, par :

· application au DVV courant (CYCLE 4) d'un pas de modulation ($\Delta$) en plus et en moins au cours de cycles cardiaques successifs respectifs (CYCLE 5, CYCLE 6), puis

· calcul dudit signal d'erreur de DVV par différence entre les valeurs respectives de signal hémodynamique mesurées avec les pas de modulation en plus et en moins, et

- les cycles d'application d'un pas de modulation au DAV et les cycles d'application d'un pas de modulation au DVV sont séquencés en alternance, le régulateur de contrôle du DVV étant inhibé pendant l'application d'un pas de modulation au DAV, et *vice versa.*

2. Le dispositif médical actif de la revendication 1, dans lequel lesdits régulateurs (36, 46) en boucle fermée de contrôle du DAV et de contrôle du DVV sont des régulateurs de type PID.

3. Le dispositif médical actif de la revendication 1, dans lequel lesdits régulateurs (36, 46) en boucle fermée de contrôle du DAV et de contrôle du DVV sont des régulateurs de type "PID tronqué" avec limitation des corrections à une valeur maximale donnée.

4. Le dispositif médical actif de la revendication 1, dans lequel lesdits régulateurs (36, 46) en boucle fermée de contrôle du DAV et de contrôle du DVV sont des régulateurs de type "PID signe" avec pas d'adaptation fixe.

5. Le dispositif médical actif de la revendication 1, dans lequel les moyens générateurs de signal d'erreur de DAV et/ou de DVV comprennent des moyens de démodulation aptes à appliquer en entrée d'un filtre dérivateur du premier ordre à deux coefficients, ou d'un filtre dérivateur du second ordre à trois coefficients, les échantillons de la valeur numérisée du signal hémodynamique mesuré, le signal d'erreur étant obtenu en sortie de ce filtre.

## Claims

1. Active medical device, comprising:

- an implantable cardiac prosthesis for resynchronization by biventricular stimulation, comprising means for detecting auricular and ventricular events and means (10) for stimulating the right and left ventricles;

- at least one sensor (18) capable of delivering a signal (Z) representative of a current haemodynamic parameter of the patient;

- means for applying to the stimulation means an atrioventricular delay DAV, counted from the start of the detection of a spontaneous or stimulated auricular event and at the end of which stimulation of the right ventricule is applied in the absence of any detected spontaneous ventricular event;

- means for applying to the stimulation means an interventricular delay DVV between the respective instances of stimulation of the right and left ventricles; and

- closed-loop control means (22), capable of continuously controlling the DAV and the DVV as a function of the haemodynamic signal delivered by the sensor, comprising:

a) DAV error signal generating means (38, 42) capable of generating, as a function of the signal delivered by said at least one sensor (18), a DAV error signal ($E_{DAV}$) representative of a deviation between the current value of the DAV and an optimum DAV value ($DAV_{opt}$) ;

b) DVV error signal generating means (48, 52) capable of generating, as a function of the signal delivered by said at least one sensor (18), a DVV error signal ($E_{DVV}$) representative of a deviation between the current value of the DVV and an optimum DVV value ($DVV_{opt}$) ;

c) a closed-loop mode regulator (36) for controlling the DAV, receiving as input said DAV error signal ($E_{DAV}$) and delivering as output a DAV signal; and

d) a closed-loop mode regulator (46) for controlling the DVV, receiving as input said DVV error signal ($E_{DVV}$) and delivering as output a DVV signal,

device **characterized in that**:

- the DAV error signal generating means (38, 42) are capable of operating, over an interval of at least three cardiac cycles, by:

• application to the current DAV (CYCLE 1) of one modulation pitch (Δ) extra and less during respective successive cardiac cycles (CYCLE 2, CYCLE 3), then
• calculating said DAV error signal by difference between the respective haemodynamic signal values measured with the modulation pitches extra and less,

- the DVV error signal generating means (48, 52) are capable of operating, over an interval of at least three cardiac cycles, by:

• application to the current DVV (CYCLE 4) of one modulation pitch (Δ) extra and less during respective successive cardiac cycles (CYCLE 5, CYCLE 6), then
• calculating said DVV error signal by difference between the respective haemodynamic signal values measured with the modulation pitches extra and less, and

- the cycles of application of a modulation pitch to the DAV and the cycles of application of a modulation pitch to the DVV are sequenced alternately, the DVV control regulator being inhibited during the application of a modulation pitch to the DAV, and *vice versa.*

2. Active medical device of claim 1, in which said closed-loop mode regulators (36, 46) for controlling the DAV and for controlling the DVV are regulators of PID type.

3. Active medical device of claim 1, in which said closed-loop mode regulators (36, 46) for controlling the DAV and for controlling the DVV are regulators of "truncated PID" type with limitation of the corrections to a given maximum value.

4. Active medical device of claim 1, in which said closed-loop mode regulators (36, 46) for controlling the DAV and for controlling the DVV are regulators of "sign PID" type with fixed adaptation pitch.

5. Active medical device of claim 1, in which the DAV and/or DVV error signal generating means comprise demodulation means capable of applying, to the input of a first order derivative filter with two coefficients, or of a second order derivative filter with three coefficients, the samples of the digitized value of the measured haemodynamic signal, the error signal being obtained at the output of this filter.

**Patentansprüche**

1. Aktive medizinische Vorrichtung, die Folgendes enthält:

- eine implantierbare Herzprothese zur Resynchronisation durch biventrikuläre Stimulation, die Einrichtungen zur Erfassung aurikulärer und ventrikulärer Ereignisse und Einrichtungen (10) zur Stimulation der rechten und linken Ventrikel aufweist;
- mindestens einen Sensor (18), der ein Signal (Z) liefern kann, das für einen laufenden hämodynamischen Parameter des Patienten repräsentativ ist;
- Einrichtungen, um an die Stimulationseinrichtungen eine atrioventrikuläre Verzögerung DAV anzuwenden, die ausgehend von der Erfassung eines spontanen oder stimulierten aurikulären Ereignisses gezählt wird und an deren Ende in Abwesenheit eines erfassten spontanen ventrikulären Ereignisses eine Stimulation des rechten Ventrikels angewendet wird;
- Einrichtungen, um an die Stimulationseinrichtungen eine interventrikuläre Verzögerung DVV zwischen den Stimulationszeitpunkten der rechten bzw. linken Ventrikel anzuwenden; und
- Regelungseinrichtungen (22), die kontinuierlich die DAV und die DVV abhängig von dem vom Sensor gelieferten hämodynamischen Signal steuern können, die Folgendes enthalten:

a) ein DAV-Fehlersignal erzeugende Einrichtungen (38, 42), die abhängig von dem vom mindestens einen Sensor (18) gelieferten Signal ein DAV-Fehlersignal ($E_{DAV}$) liefern können, das für eine Abweichung zwischen dem laufenden Wert der DAV und einem optimalen DAV-Wert ($DAV_{opt}$) repräsentativ ist;

b)ein DVV-Fehlersignal erzeugende Einrichtungen (48, 52), die abhängig von dem vom mindestens einen Sensor (18) gelieferten Signal ein DVV-Fehlersignal ($E_{DVV}$) liefern können, das für eine Abweichung zwischen dem laufenden Wert der DVV und einem optimalen DVV-Wert ($DVV_{opt}$) repräsentativ ist;
c)einen Rückkopplungsregler (36) zur Steuerung der DAV, der am Eingang das DAV-Fehlersignal ($E_{DAV}$) empfängt und am Ausgang ein DAV-Signal liefert; und
d)einen Rückkopplungsregler (46) zur Steuerung der DVV, der am Eingang das DVV-Fehlersignal ($E_{DVV}$) empfängt und am Ausgang ein DVV-Signal liefert,

wobei die Vorrichtung **dadurch gekennzeichnet ist, dass**:

- die ein DAV-Fehlersignal erzeugenden Einrichtungen (38, 42) über einen Zeitraum von mindestens drei Herzzyklen arbeiten können durch:

. Anwendung an die laufende DAV (ZYKLUS 1) eines Modulationsschritts mehr und eines Modulationsschritts weniger ($\Delta$) während aufeinanderfolgender Herzzyklen (ZYKLUS 2, ZYKLUS 3), dann
. Berechnung des DAV-Fehlersignals durch Differenz zwischen den jeweiligen Werten eines hämodynamischen Signals, die mit den mehr oder weniger Modulationsschritten gemessen werden,

- die ein DVV-Fehlersignal erzeugenden Einrichtungen (48, 52) über einen Zeitraum von mindestens drei Herzzyklen arbeiten können durch:

. Anwendung an die laufende DVV (ZYKLUS 4) eines Modulationsschritts mehr oder eines Modulationsschritts weniger ($\Delta$) während aufeinanderfolgender Herzzyklen (ZYKLUS 5, ZYKLUS 6), dann
. Berechnung des DVV-Fehlersignals durch Differenz zwischen den jeweiligen Werten eines hämodynamischen Signals, die mit den mehr oder weniger Modulationsschritten gemessen werden, und

- die Anwendungszyklen eines Modulationsschritts an die DAV und die Anwendungszyklen eines Modulationsschritts an die DVV abwechselnd sequenziert werden, wobei der Steuerregler der DVV während der Anwendung eines Modulationsschritts an die DAV gesperrt wird und umgekehrt.

2. Aktive medizinische Vorrichtung nach Anspruch 1, wobei die Rückkopplungsregler (36, 46) zur Steuerung der DAV und zur Steuerung der DVV Regler von der Art PID sind.

3. Aktive medizinische Vorrichtung nach Anspruch 1, wobei die Rückkopplungsregler (36, 46) zur Steuerung der DAV und zur Steuerung der DVV Regler von der Art "gekürzte PID" mit Begrenzung der Korrekturen auf einen gegebenen maximalen Wert sind.

4. Aktive medizinische Vorrichtung nach Anspruch 1, wobei die Rückkopplungsregler (36, 46) zur Steuerung der DAV und zur Steuerung der DVV Regler von der Art "Vorzeichen-PID" mit festem Anpassungsschritt sind.

5. Aktive medizinische Vorrichtung nach Anspruch 1, wobei die ein DAV- und/oder DVV-Fehlersignal erzeugenden Einrichtungen Demodulationseinrichtungen enthalten, die am Eingang eines Abzweigungsfilters erster Ordnung mit zwei Koeffizienten oder eines Abzweigungsfilters zweiter Ordnung mit drei Koeffizienten die Tastproben des digitalen Werts des gemessenen hämodynamischen Signals anlegen können, wobei das Fehlersignal am Ausgang dieses Filters erhalten wird.

Fig. 1

Fig. 4

Fig. 5

Fig. 2

Fig. 3

Fig. 6

Fig. 7

**Fig. 8**

**Fig. 9**

**Fig. 10**

Fig. 11

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1108446 A1
- US 6792310 B1
- US 20070066905 A1
- US 20040078058 A1
- WO 200090397 A2
- WO 2006126185 A2
- WO 2008010220 A
- EP 0515319 A1
- EP 0655260 A1
- EP 1116497 A1
- EP 1138346 A1
- EP 1741387 A1

**Littérature non-brevet citée dans la description**

- **JM DUPUIS et al.** Programming Optimal Atrioventricular Delay in Dual Chamber Pacing Using Peak Endocardial Acceleration : Comparison with a Standard Echocardiographic Procedure. *PACE,* 2003, vol. 26, 210-213
- **DAVID TAMBORERO et al.** Optimization of the Interventricular Delay in Cardiac Resynchronization Therapy Using the QRS Width. *American Journal of Cardiology,* 15 Novembre 2009, vol. 104 (10), 1407-1412